# EUROPEAN PATENT APPLICATION

(11) **EP 4 032 588 A1**
(43) Date of publication of application: **27.07.2022**
(21) Application number: 20865812.0
(22) Date of filing: 11.09.2020
(51) Int. Cl.: A61P 25/16, A61K 9/70, A61K 47/12, A61K 31/4045

(54) **ROPINIROLE-CONTAINING PATCH AND METHOD FOR IMPROVING SKIN PERMEABILITY OF ROPINIROLE**

(30) Priority: 18.09.2019 JP 2019168963
(71) Applicant: Hisamitsu Pharmaceutical Co., Inc., Tosu-shi, Saga 841-0017 (JP)
(72) Inventor: UCHIDA Naoyuki, Tsukuba-shi, Ibaraki 305-0856 (JP); AMANO Satoshi, Tsukuba-shi, Ibaraki 305-0856 (JP)
(74) Representative: Grünecker Patent- und Rechtsanwälte PartG mbB
(86) International application number: PCT/JP2020/034464
(87) International publication number: WO 2021/054257

(57) **Abstract**

A ropinirole-containing patch comprising an adhesive agent layer and a backing layer, wherein
the adhesive agent layer containing:
at least one selected from the group consisting of ropinirole and a pharmaceutically acceptable salt thereof;
a fatty acid metal salt having 7 or more carbon atoms; and
an adhesive agent.

## Description

### [Technical Field]

The present invention relates to a patch and a method for improving skin permeability of a drug, and more specifically to a patch comprising ropinirole and/or a pharmaceutically acceptable salt thereof and a method for improving skin permeability of ropinirole and/or a pharmaceutically acceptable salt thereof.

### [Background Art]

Ropinirole is known as a drug useful for treating Parkinson's disease, restless legs syndrome, and the like, and transdermal administration of a preparation containing ropinirole and/or a pharmaceutically acceptable salt thereof has been studied in recent years from the viewpoint of reduction in frequency of administration, improvement in the compliance, and easiness of administration and discontinuation thereof.

For example, International Publication No. WO2010/134433 (PTL 1) states a preparation for transdermal absorption comprising a backing and an adhesive agent layer containing ropinirole or a pharmaceutically acceptable acid addition salt thereof, and International Publication No. WO2012/165253 (PTL 2) and International Publication No. WO2012/165254 (PTL 3) each state a ropinirole-containing patch comprising a backing layer and an adhesive agent layer containing ropinirole and/or a pharmaceutically acceptable salt thereof.

In addition, International Publication No. WO2009/107478 (PTL 4) and International Publication No. WO2009/107479 (PTL 5) each state a patch comprising a backing and an adhesive agent layer, wherein the adhesive agent layer contains ropinirole and a metal salt generated by reaction of an acid addition salt of ropinirole and a desalting agent.

In transdermal administration of a preparation containing ropinirole and/or a pharmaceutically acceptable salt thereof, excellent skin permeability is required. PTLs 1 to 5 state that absorption enhancers for enhancing transdermal absorption of ropinirole and/or a pharmaceutically acceptable salt thereof may be used, and state, for example, aliphatic alcohols, fatty acids, and the like as the absorption enhancers. In addition, as stated in PTLs 4 to 5, it is also known to cause free ropinirole (free form) to be transdermally absorbed by desalting an acid addition salt of ropinirole with a desalting agent during production of the preparation or in the produced preparation, and PTLs 1 to 5 state, for example, sodium hydroxide and the like as the desalting agent. However, besides the configuration using these absorption enhancers and the configuration using a desalting agent such as sodium hydroxide, a method for improving skin permeability of ropinirole and/or a pharmaceutically acceptable salt thereof has not been sufficiently studied yet.

### [Citation List]

### [Patent Literature]

[PTL 1] International Publication No. WO2010/134433
[PTL 2] International Publication No. WO2012/165253
[PTL 3] International Publication No. WO2012/165254
[PTL 4] International Publication No. WO2009/107478
[PTL 5] International Publication No. WO2009/107479

### [Summary of Invention]

### [Technical Problem]

The present invention has been made in view of the above problems of the conventional techniques, and an object thereof is to provide a ropinirole-containing patch that is excellent in skin permeability of ropinirole and/or a pharmaceutically acceptable salt thereof and a method for improving skin permeability of ropinirole and/or a pharmaceutically acceptable salt thereof, from a viewpoint different from the configurations of the conventional ropinirole-containing patches.

### [Solution to Problem]

The present inventors continuously conducted earnest studies in order to achieve the above object, and consequently have found that in a patch comprising a backing layer and an adhesive agent layer, wherein the adhesive agent layer containing: at least one selected from the group consisting of ropinirole and a pharmaceutically acceptable salt thereof; and an adhesive agent, it is possible to obtain a patch excellent in skin permeability of ropinirole and/or a pharmaceutically acceptable salt thereof by causing the adhesive agent layer to further contain a fatty acid metal salt having 7 or more carbon atoms. The present inventors have also found that even in the case of being combined with an acid addition salt of ropinirole, such excellent skin permeability is not an effect expected from mere desalting, that is, an effect exhibited by the fatty acid metal salt reacting as a desalting agent for an acid addition salt of ropinirole to generate a fatty acid functioning as an absorption enhancer, but is surprisingly an effect exhibited differently by using the specific fatty acid metal salt although the reason is unclear, and have led to the completion of the present invention.

Specifically, a ropinirole-containing patch of the present invention comprises a backing layer and an adhesive agent layer, wherein
the adhesive agent layer containing:
at least one selected from the group consisting of ropinirole and a pharmaceutically acceptable salt thereof;
a fatty acid metal salt having 7 or more carbon atoms; and
an adhesive agent.

In the ropinirole-containing patch of the present invention, the fatty acid metal salt is preferably a fatty acid alkali metal salt, and also the fatty acid metal salt is preferably a metal salt of at least one fatty acid selected from the group consisting of caprylic acid, oleic acid, lauric acid, stearic acid, capric acid, myristic acid, and palmitic acid.

Moreover, in the ropinirole-containing patch of the present invention, the adhesive agent is preferably at least one selected from the group consisting of a rubber-based adhesive agent, an acrylic-based adhesive agent having no carboxy group, and a silicone-based adhesive agent.

In addition, in the ropinirole-containing patch of the present invention, a content of the fatty acid metal salt is preferably 0.1 to 45% by mass relative to a total mass of the adhesive agent layer, and in the adhesive agent layer, a content of the fatty acid metal salt is preferably 0.1 to 5.0 mol relative to 1.0 mol of the at least one selected from the group consisting of ropinirole and a pharmaceutically acceptable salt thereof in content in terms of free ropinirole.

Moreover, in the ropinirole-containing patch of the present invention, a content of the at least one selected from the group consisting of ropinirole and a pharmaceutically acceptable salt thereof in terms of free ropinirole is preferably 5 to 30% by mass relative to a total mass of the adhesive agent layer.

A method for improving skin permeability of ropinirole of the present invention is a method for improving skin permeability of at least one selected from the group consisting of ropinirole and a pharmaceutically acceptable salt thereof in a ropinirole-containing patch comprising a backing layer and an adhesive agent layer, wherein
the adhesive agent layer containing:
the at least one selected from the group consisting of ropinirole and a pharmaceutically acceptable salt thereof and
an adhesive agent,
the method comprising:
   a step of causing the adhesive agent layer to further contain a fatty acid metal salt having 7 or more carbon atoms.

### [Advantageous Effects of Invention]

The present invention makes it possible to provide a ropinirole-containing patch that is excellent in skin permeability of ropinirole and/or a pharmaceutically acceptable salt thereof and a method for improving skin permeability of ropinirole and/or a pharmaceutically acceptable salt thereof.

### [Description of Embodiments]

Hereinafter, the present invention will be described in detail based on preferred embodiments.

A ropinirole-containing patch of the present invention (hereinafter sometimes simply referred to as a "patch of the present invention") comprises a backing layer and an adhesive agent layer, wherein
the adhesive agent layer containing:
at least one selected from the group consisting of ropinirole and a pharmaceutically acceptable salt thereof;
a fatty acid metal salt having 7 or more carbon atoms; and
an adhesive agent.

The patch of the present invention comprises the backing layer and the adhesive agent layer, and preferably, the adhesive agent layer is arranged on one surface of the backing layer.

The backing layer according to the present invention is not particularly limited as long as the backing layer can support the adhesive agent layer described later, and a publicly-known backing layer for patches may be employed as appropriate. The material of the backing layer according to the present invention includes, for example, polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate (PET), polybutylene terephthalate, and polyethylene naphthalate; cellulose derivatives; synthetic resins such as polyurethane, and metals such as aluminum. Among these, polyester is preferable from the viewpoint of non-drug adsorbing property and non-drug permeability. The form of the backing layer includes, for example, films; sheets such as a sheet, a sheet-shaped porous body, and a sheet-shaped foam; fabrics such as a woven fabric, a braided fabric, and a nonwoven fabric; foils; and laminates of these. In addition, the thickness of the backing layer is not particularly limited, but is preferably within a range of 5 to 1000 µm from the viewpoint of easiness of work in applying the patch and easiness of production.

The patch of the present invention may further comprise a release liner on a surface of the adhesive agent layer opposite to the backing layer. Such a release liner includes a film and a sheet made of a material such as polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate; cellulose derivatives; synthetic resins such as polyurethane, aluminum, and paper, and a laminate of these. These release liners are preferably those processed by a release treatment using a silicone-containing compound coating, fluorine-containing compound coating, or the like on a surface thereof which comes into contact with the adhesive agent layer so that the release liner can be easily peeled off the adhesive agent layer.

The adhesive agent layer according to the present invention contains at least one selected from the group consisting of ropinirole and a pharmaceutically acceptable salt thereof (in the Specification, sometimes referred to as "ropinirole and/or a pharmaceutically acceptable salt thereof") as the drug.

Ropinirole contained in the adhesive agent layer according to the present invention may be a free form (in the Specification, sometimes referred to as "free ropinirole") or may be a pharmaceutically acceptable salt thereof, or a free ropinirole obtained by desalting a pharmaceutically acceptable salt of ropinirole in a preparation during the production and/or after the production, and one of these may be used alone or a mixture of two or more of these may be used.

As the pharmaceutically acceptable salt of ropinirole, a pharmaceutically acceptable acid addition salt of ropinirole (in the Specification, sometimes referred to as a "ropinirole acid addition salt") is preferable. The acid of the ropinirole acid addition salt includes, for example, hydrochloric acid, sulfuric acid, nitric acid, phosphoric acid, phosphorous acid, hydrobromic acid, maleic acid, malic acid, ascorbic acid, tartaric acid, and fumaric acid.

When the adhesive agent layer is caused to contain the ropinirole acid addition salt as an ingredient of the patch of the present invention, since a fatty acid metal salt described below is also a weak base, when the ropinirole acid addition salt is mixed with the fatty acid metal salt, all or part of the ropinirole acid addition salt is desalted (neutralized) in the adhesive agent layer of the preparation during the production and/or after the production, so that free ropinirole in the state of a free base is obtained, and consequently, it becomes possible to cause free ropinirole having a higher tissue absorption to exist in the adhesive agent layer when the preparation is applied.

The content of the ropinirole and/or a pharmaceutically acceptable salt thereof according to the present invention (the content of free ropinirole or the content of a pharmaceutically acceptable salt of ropinirole, or in a case where both of free ropinirole and a pharmaceutically acceptable salt of ropinirole are contained, the total content of these, the same applies hereinafter.) is preferably 5 to 30% by mass, more preferably 5 to 25% by mass, and further preferably 5 to 20% by mass, and even more preferably 5 to 13.2% by mass relative to the total mass of the adhesive agent layer in terms of free ropinirole. If the content of the ropinirole and/or a pharmaceutically acceptable salt thereof is less than the lower limit, the skin permeability of the ropinirole and/or a pharmaceutically acceptable salt thereof (hereinafter, sometimes referred to as "skin permeability of ropinirole") tends to decrease. On the other hand, if the content is more than the upper limit, since the absolute amount in the adhesive agent layer is increased, this tends to make it difficult to apply the composition in the adhesive agent layer when the adhesive agent layer is formed, and make it difficult to obtain a uniform preparation.

The adhesive agent layer according to the present invention contains a fatty acid metal salt having 7 or more carbon atoms (in the Specification, the "fatty acid metal salt" is synonymously used unless otherwise noted). The fatty acid metal salt according to the present invention is one in which a monovalent carboxylic acid (fatty acid) of a long-chain hydrocarbon having 7 or more carbon atoms and a metal ion are ionically bound.

The fatty acid metal salt according to the present invention needs to have 7 or more carbon atoms . The number of carbon atoms of the fatty acid metal salt is preferably 8 or more, more preferably 8 to 22, further preferably 10 or more, even more preferably 10 to 22, particularly preferably 10 to 20, and most preferably 10 to 18. If the number of carbon atoms is less than the lower limit, the skin permeability of ropinirole decreases in some cases. In addition, if the number of carbon atoms is more than the upper limit as well, the skin permeability of ropinirole tends to decrease.

The fatty acid forming the fatty acid metal salt according to the present invention may be saturated or unsaturated, and may be linear or have a branched chain, is preferably at least one selected from the group consisting of oleic acid, lauric acid, stearic acid, capric acid, myristic acid, palmitic acid, caprylic acid, linoleic acid, and behenic acid, more preferably at least one selected from the group consisting of caprylic acid, oleic acid, lauric acid, stearic acid, capric acid, myristic acid, and palmitic acid, and further preferably at least one selected from the group consisting of caprylic acid, oleic acid, lauric acid, stearic acid, and capric acid. In addition, the metal forming the fatty acid metal salt according to the present invention is preferably at least one selected from the group consisting of alkali metals and alkaline-earth metals, more preferably at least one selected from the group consisting of alkali metals, further preferably at least one selected from the group consisting of sodium and potassium, and even more preferably sodium.

More specifically, the fatty acid metal salt according to the present invention includes sodium caprylate, potassium caprylate, sodium oleate, potassium oleate, lithium oleate, sodium myristate, potassium myristate, lithium myristate, sodium laurate, potassium laurate, sodium stearate, potassium stearate, lithium stearate, sodium caprate, potassium caprate, lithium caprate, sodium palmitate, potassium palmitate, and the like, and one of these may be used alone or two or more of these may be used in combination. Among these, the fatty acid metal salt according to the present invention is preferably at least one selected from the group consisting of sodium caprylate, sodium oleate, sodium caprate, sodium laurate, sodium stearate, and potassium stearate, more preferably at least one selected from the group consisting of sodium caprylate, sodium oleate, sodium caprate, sodium laurate, and potassium stearate, and even more preferably at least one selected from the group consisting of sodium oleate, sodium caprate, and sodium laurate.

The content of the fatty acid metal salt according to the present invention (which is, in the case where there are two or more, the total content of these, the same applies hereinafter.) is preferably 0.1 to 45% by mass, more preferably 0.1 to 40% by mass, further preferably 1 to 30% by mass, and even more preferably 5 to 20% by mass, relative to the total mass of the adhesive agent layer. If the content of the fatty acid metal salt is less than the lower limit, the skin permeability of ropinirole tends to decrease. On the other hand, if the content is more than the upper limit, the cohesive force of the adhesive agent layer tends to decrease. Note that in a case where a component derived from the fatty acid metal salt described below is contained, the content of the fatty acid metal salt according to the present invention also contains the content of the component in terms of fatty acid metal salt in addition to the content of the fatty acid metal salt.

In addition, in the adhesive agent layer according to the present invention, the content of the fatty acid metal salt is, in terms of fatty acid (in terms of the number of carboxy group residues (COO)), preferably 0.1 to 5.0 mol, more preferably 0.2 to 4.0 mol, further preferably 0.5 to 3.0 mol, and even more preferably 0.6 to 3.0 mol, relative to 1.0 mol of the ropinirole and/or a pharmaceutically acceptable salt thereof in content in terms of free ropinirole. If the content of the fatty acid metal salt is less than the lower limit, the skin permeability of ropinirole tends to decrease. On the other hand, if the content is more than the upper limit, the cohesive force of the adhesive agent layer tends to decrease.

In a case where the adhesive agent layer is caused to contain the ropinirole acid addition salt as an ingredient of the patch of the present invention, all or part of the ropinirole acid addition salt is desalted in the adhesive agent layer of the preparation during the production and/or after the production by the fatty acid metal salt, which is also a weak base. Hence, in this case, the adhesive agent layer according to the present invention only has to contain at least a mixture of the ropinirole acid addition salt and the fatty acid metal salt besides the adhesive agent described below, and may contain, as the mixture, a component derived from free ropinirole and the fatty acid metal salt as a desalting reaction product of the ropinirole acid addition salt and the fatty acidmetal salt (component derived from the fatty acid metal salt).

The component derived from the fatty acid metal salt includes fatty acids, fatty acid ions, metal ions, and metal salts, and one of these may be used alone or two or more of these may be used in combination. Although it depends on the acid of the ropinirole acid addition salt or the type of an acid which may be contained in the adhesive agent layer as necessary, the metal salt includes, for example, metal salts with hydrochloric acid, sulfuric acid, nitric acid, phosphoric acid, phosphorous acid, hydrobromic acid, maleic acid, malic acid, ascorbic acid, tartaric acid, and fumaric acid.

The adhesive agent layer according to the present invention contains an adhesive agent. The adhesive agent according to the present invention includes a rubber-based adhesive agent, an acrylic-based adhesive agent, a silicone-based adhesive agent, and the like, and one of these may be used alone or two or more of these may be used in combination. Among these, the adhesive agent according to the present invention is preferably at least one selected from the group consisting of a rubber-based adhesive agent, an acrylic-based adhesive agent having no carboxy group, and a silicone-based adhesive agent and more preferably at least one selected from the group consisting of a rubber-based adhesive agent and an acrylic-based adhesive agent having no carboxy group, and further preferably contains at least a rubber-based adhesive agent.

The content of the adhesive agent in the adhesive agent layer according to the present invention (which is, in the case where there are two or more, the total content of these, the same applies hereinafter.) is preferably 10 to 90% by mass, and more preferably 20 to 70% by mass, relative to the total mass of the adhesive agent layer.

The rubber-based adhesive agent according to the present invention includes natural rubber and synthetic rubbers, and more preferably is at least one selected from the group consisting of synthetic rubbers having no hydroxyl group or carboxy group such as styrene-isoprene-styrene block copolymer (SIS), isoprene rubber, polyisobutylene (PIB), styrene-butadiene-styrene block copolymer (SBS), styrene-butadiene rubber (SBR), and polybutene from the viewpoint that these tend to be excellent in the cohesive force of the adhesive agent layer and the skin permeability of ropinirole. In the present invention, the "synthetic rubbers having no hydroxyl group or carboxy group" represent synthetic rubbers having substantially no hydroxyl group or carboxy group, and preferably those in each of which the content of the hydroxyl group and the carboxy group in the molecule is less than 3% by mass.

In the case where the adhesive agent layer according to the present invention contains the rubber-based adhesive agent, the content of the rubber-based adhesive agent (which is, in the case where there are two or more, the total content of these, the same applies hereinafter.) is preferably 10 to 80% by mass, more preferably 10 to 70% by mass, further preferably 10 to 55% by mass, and even more preferably 15 to 35% by mass relative to the total mass of the adhesive agent layer. If the content of the rubber-based adhesive agent is less than the lower limit, the cohesive force of the adhesive agent layer tends to decrease. On the other hand, if the content is more than the upper limit, the adhesive agent layer becomes so hard that the adhesiveness of the patch tends to decrease.

The acrylic-based adhesive agent according to the present invention includes those listed as adhesive agents in "Japanese Pharmaceutical Excipients Directory 2016 (edited by International Pharmaceutical Excipients Council Japan)". One of these may be used alone or two or more of these may be used in combination, but the acrylic-based adhesive agent is preferably an acrylic-based adhesive agent having no carboxy group, and more preferably an acrylic-based adhesive agent having no functional group. In the present invention, the "acrylic-based adhesive agent having no carboxy group" and the "acrylic-based adhesive agent having no functional group" represent acrylic-based polymers having substantially no carboxy group or functional group, and preferably those in each of which the content of the carboxy group and the functional group in the polymer is less than 3% by mass.

The acrylic-based adhesive agent having no carboxy group includes, for example, acrylic-based adhesive agents that have substantially no functional group such as 2-ethylhexyl acrylate·vinylpyrrolidone copolymer, 2-ethylhexyl acrylate·2-ethylhexyl methacrylate·dodecyl methacrylate copolymer, 2-ethylhexyl acrylate·vinyl acetate copolymer, 2-ethylhexyl acrylate·methyl methacrylate·butyl acrylate copolymer, 2-ethylhexyl acrylate·methacrylic acid copolymer, and ethyl acrylate·methyl methacrylate copolymer; and acrylic-based adhesive agents that have a hydroxy group such as 2-ethylhexyl methacrylate·vinyl acetate·2-hydroxyethyl acrylate copolymer, 2-hydroxyethyl methacrylate copolymer, 2-hydroxypropyl methacrylate copolymer, 3-hydroxypropyl methacrylate copolymer, 4-hydroxybutyl methacrylate copolymer, and 2-ethylhexyl acrylate·vinyl acetate·hydroxyethyl acrylate·glycidyl methacrylate copolymer. One of these may be used alone or two or more of these may be used in combination.

As the acrylic-based adhesive agent having no carboxy group, a commercially-available one may be used as appropriate, and it is possible to use as appropriate, for example, acrylic-based polymers contained in MAS 811 and MAS 683 (manufactured by CosMED Pharmaceutical Co. Ltd.); 87-900A, 87-901A, 87-9301, 87-4098, 87-9088, and 87-9085 of Duro-Tak (registered trademark) acrylic adhesive agent series (manufactured by Henkel AG & Co. KGaA); GMS 3083, GMS 3253, and GMS 3235 of GELVA (registered trademark) acrylic adhesive agent series (manufactured by Henkel AG & Co. KGaA), and the like, and acrylic-based polymers contained in 87-202A, 87-2287, 87-2516, 87-2510, 87-4287, 87-2525, 87-201A, 87-202A, 87-208A, 87-502A, 87-503A, and 87-504A of Duro-Tak (registered trademark) acrylic adhesive agent series (manufactured by Henkel AG & Co. KGaA); GMS 788 and GMS 737 of GELVA (registered trademark) acrylic adhesive agent series (manufactured by Henkel AG & Co. KGaA), and the like.

In the case where the adhesive agent layer according to the present invention contains an acrylic-based adhesive agent, the content of the acrylic-based adhesive agent (which is, in the case where there are two or more, the total content of these, the same applies hereinafter.) is preferably 10 to 90% by mass, more preferably 10 to 80% by mass, and further preferably 20 to 70% by mass, relative to the total mass of the adhesive agent layer. If the content of the acrylic-based adhesive agent is less than the lower limit, the cohesive force of the adhesive agent layer tends to decrease. On the other hand, if the content is more than the upper limit, the skin permeability of ropinirole tends to decrease.

The silicone-based adhesive agent according to the present invention includes silicone rubbers represented by MQ (polydimethylsiloxane), VMQ (polymethylvinylsiloxane), PMQ (polymethylphenylsiloxane), and PVMQ (polyphenylvinylmethylsiloxane) in ASTM standard (ASTM D 1418), a mixture of at least one of these and a silicone resin such as polyditrimethylsilylsiloxane other than silicone rubbers, and the like. One of these may be used alone or two or more of these may be used in combination. Note that in the case where a silicone resin other than the silicone rubbers is mixed, the content of the silicone resin is preferably 0.1 to 20% by mass relative to the total mass of the silicone-based adhesive agent.

In addition, as these silicone-based adhesive agents, commercially-available ones may be used as appropriate. For example, silicone-based adhesive agents provided by Dow Corning Corp. under the following model numbers: BIO-PSA-7-410X, BIO-PSA-7-420X, BIO-PSA-7-430X, BIO-PSA-7-440X, BIO-PSA-7-450X, BIO-PSA-7-460X (the Xs are each independently 1 or 2), BIO-PSA AC7-4201, BIO-PSA AC7-4301, BIO-PSA AC7-4302, MD7-4502, MD7-4602, 7-9700, MG7-9800, MG7-9850 ; BIO-PSA-7-4560, which is a hot melt silicone adhesive agent, and the like may be used as appropriate.

Moreover, the silicone-based adhesive agent according to the present invention may be, for example, in the case of having methyl groups, one obtained by dehydrogenating the methyl groups to remove hydrogen atoms to crosslink the methyl groups by blending a peroxide; in the case of having vinyl groups, one obtained by binding a crosslinking agent composed of a SiH group-containing siloxane compound to crosslink the vinyl groups; in the case of having hydroxyl groups (that is, in the case of having silanol groups), one obtained by crosslinking the silanol groups through dehydration condensation, or the like.

In the case where the adhesive agent layer according to the present invention contains the silicone-based adhesive agent, the content of the silicone-based adhesive agent (which is, in the case where there are two or more, the total content of these, the same applies hereinafter.) is preferably 10 to 90% by mass, more preferably 10 to 80% by mass, and further preferably 20 to 70% by mass relative to the total mass of the adhesive agent layer. If the content of the silicone-based adhesive agent is less than the lower limit, the cohesive force of the adhesive agent layer tends to decrease. On the other hand, if the content is more than the upper limit, the skin permeability of ropinirole tends to decrease.

The adhesive agent layer according to the present invention may further contain another drug other than ropinirole and a pharmaceutically acceptable salt thereof; an absorption enhancer (transdermal absorption enhancer) ; an excipient such as an adsorbent, a tackifier, a plasticizer, a solubilizer for the drug, a filler, a stabilizer, a preservative, or the like as long as the effect of the present invention is not hindered.

The other drug other than ropinirole and a pharmaceutically acceptable salt thereof includes, for example, non-steroidal anti-inflammatory drugs (diclofenac, indomethacin, ketoprofen, felbinac, loxoprofen, ibuprofen, flurbiprofen, tiaprofenic acid, acemetacin, sulindac, etodolac, tolmetin, piroxicam, meloxicam, ampiroxicam, naproxen, azapropazone, methyl salicylate, glycol salicylate, valdecoxib, celecoxib, rofecoxib, amfenac, and the like), anti-histamine drugs (diphenhydramine, chlorphenamine, mequitazin, homochlorcyclizine, and the like), anti-hypertensive drugs (diltiazem, nicardipine, nilvadipine, metoprolol, bisoprolol, trandolapril, and the like), anti-Parkinson drugs (pergolide, bromocriptine, selegiline, and the like), bronchodilators (tulobuterol, isoproterenol, salbutamol, and the like), anti-allergic drugs (ketotifen, loratadine, azelastine, terfenadine, cetirizine, acitazanolast, and the like), local anesthetics (lidocaine, dibucaine, and the like), anesthetic-based analgesics (morphine and the like), agents for urinary organs (oxybutynin, tamsulosin, and the like), neuropsychiatric drugs (promazine, chlorpromazine, and the like), steroid hormone drugs (estradiol, progesterone, norethisterone, cortisone, hydrocortisone, and the like), antidepressants (sertraline, fluoxetine, paroxetin, citalopram, and the like), anti-dementia drugs (donepezil, rivastigmine, galantamine, and the like), anti-psychotic drugs (risperidone, olanzapine, and the like), central nervous system stimulant drugs (methylphenidate and the like), osteoporosis treatment drugs (raloxifene, alendronate, and the like), breast cancer preventive drugs (tamoxifen and the like), anti-obesity drugs (mazindol, sibutramine, and the like), insomnia treatment drugs (melatonin and the like), and anti-rheumatic drugs (actarit and the like). One of these may be used alone or two or more of these may be used in combination.

The absorption enhancer includes, for example, isopropyl myristate, isopropyl palmitate, lauryl alcohol, hexyl laurate, myristyl alcohol, oleyl alcohol, isostearyl alcohol, octyldodecanol, benzyl alcohol, glycerine monooleate (GMO), propylene glycol monolaurate (PGML), polyoxyethylene sorbitan monooleate (Tween 80), polyoxyethylene sorbitan tristearate (Tween 65), polyoxyethylene sorbitan monostearate (Tween 60), polyoxyethylene sorbitan monolaurate (Tween 20), lauric acid diethanolamide (LADA), and the like. One of these may be used alone or two or more of these may be used in combination.

The adsorbent includes inorganic and/or organic substances having moisture absorbency, and more specifically includes minerals such as talc, kaolin, and bentonite; fumed silica (Aerosil (registered trademark) and the like), silicon compounds such as hydrous silica; metal compounds such as zinc oxide and dried aluminum hydroxide gel; weak acids such as lactic acid and acetic acid; sugars such as dextrin; and high molecular polymers such as polyvinylpyrrolidone, aminoalkyl methacrylate copolymer, crospovidone, carboxy vinyl polymer, and butyl methacrylate-methyl methacrylate copolymer. One of these may be used alone or two or more of these may be used in combination.

The tackifier is blended mainly for the purpose of enhancing the adhesiveness of the adhesive agent. Such a tackifier includes, for example, rosin-based resins, terpene-based resins, petroleum-based resins (alicyclic saturated hydrocarbon resin and the like), phenolic resins, and xylene-based resins. One of these may be used alone or two or more of these may be used in combination. In the case where such a tackifier is further contained in the adhesive agent layer, the content of the tackifier (which is, in the case where there are two or more, the total content of these) is more preferably 10 to 80% by mass, and further preferably 20 to 60% by mass, relative to the total mass of the adhesive agent layer from the viewpoint of improving the adhesive force of the adhesive agent layer and/or alleviating local irritation at the peeling-off.

The plasticizer is blended mainly for the purpose of adjusting the adhesiveness of the adhesive agent layer, the fluidity in the production of the adhesive agent layer, the transdermal absorption feature of the drug, and the like. Such a plasticizer includes, for example, silicone oil; petroleum-based oils such as paraffinic process oils (liquid paraffin and the like), naphthenic process oils, and aromatic process oils; squalane, squalene; vegetable-based oils such as olive oil, camellia oil, castor oil, tall oil, and peanut oil; dibasic esters such as dibutyl phthalate and dioctyl phthalate; liquid rubbers such as liquid polybutene and liquid isoprene rubber; diethylene glycol, polyethylene glycol, propylene glycol, dipropylene glycol, and the like. One of these may be used alone or two or more of these may be used in combination. Among these, the plasticizer is preferably at least one selected from the group consisting of silicone oil, liquid paraffin, and liquid polybutene. In the case where the plasticizer is further contained in the adhesive agent layer, the content of the plasticizer (which is, in the case where there are two or more, the total content of these) is more preferably 5 to 60% by mass, and further preferably 7 to 40% by mass, relative to the total mass of the adhesive agent layer from the viewpoint that the adhesive force as the patch becomes more favorable.

The solubilizer is blended mainly for the purpose of facilitating the dissolution of the drug. Such a solubilizer includes, for example, organic acids such as acetic acid, aliphatic alcohols, and surfactants. One of these may be used alone or two or more of these may be used in combination. Among these, the solubilizer is preferably at least one selected from the group consisting of organic acids and aliphatic alcohols.

The filler is blended mainly for the purpose of adjusting the adhesive force of the adhesive agent layer. Such a filler includes, for example, aluminum hydroxide, calcium carbonate, magnesium carbonate; silicates such as aluminum silicate and magnesium silicate; silicic acid, barium sulfate, calcium sulfate, calcium zincate, zinc oxide, and titanium oxide. One of these may be used alone or two or more of these may be used in combination.

For the adhesive agent layer according to the present invention, a component that functions as a desalting agent for the pharmaceutically acceptable acid addition salt of ropinirole besides the fatty acid metal salt according to the present invention may further be used as long as the effect of the present invention is not hindered. Such a component includes, for example, metal ion-containing desalting agents and basic nitrogen atom-containing desalting agents. The metal ion-containing desalting agent includes sodium acetate (including anhydrous sodium acetate), sodium hydroxide, potassium hydroxide, magnesium hydroxide, sodium bicarbonate, potassium bicarbonate, sodium citrate, and sodium lactate. One of these may be used alone or two or more of these may be used in combination. Since in the present invention, the patch is excellent in the skin permeability of ropinirole even without blending these components, it is preferable that such components are not substantially blended. More specifically, the amount of the component to be blended (which is, in the case where there are two or more, the total amount of these to be blended) is preferably 1% by mass or less, and more preferably 0.1% by mass or less, relative to the total mass of the adhesive agent layer.

The thickness of the adhesive agent layer according to the present invention is not particularly limited, but is preferably a thickness with which the mass per unit area of the adhesive agent layer becomes 25 to 250 g/m², more preferably a thickness with which the mass per unit area becomes 50 to 200 g/m², further preferably a thickness with which the mass per unit area becomes 50 to 150 g/m², and even more preferably a thickness with which the mass per unit area becomes 50 to 120 g/m², for example.

The patch of the present invention may be packaged (preferably sealed) in a package container for a period after the production to the time of use. The package container is not particularly limited, and a package container that can normally be used as a package container for patches may be used as appropriate. For example, it is preferable to use a plastic packaging bag, a plastic packaging bag in which a metal layer (for example, an aluminum layer) is formed, a metal packaging bag (for example, an aluminum packaging bag), and the like.

In addition, the packaged formulation in which the patch of the present invention is packaged in the package container may further have oxygen absorbing means. The oxygen absorbing means includes an oxygen absorber using an iron powder and a oxygen absorber containing vitamin C as a main component to be sealed in the package container (more specifically, AGELESS series (manufactured by Mitsubishi Gas Chemical Company, Inc.), PharmaKeep series (manufactured by Mitsubishi Gas Chemical Company, Inc.), and the like) ; and the package container including a layer having an oxygen absorbing function (more specifically, a layer in which a powder of aluminum, zinc, manganese, copper, iron, hydrosulfite, activated carbon, or the like is mixed, or the like).

The patch of the present invention may be produced, for example, by the following production method. First, the ropinirole and/or a pharmaceutically acceptable salt thereof, the fatty acid metal salt, and the adhesive agent, as well as another drug, the absorption enhancer, the excipient, the solvent, and the like as necessary are mixed in accordance with a conventional method to obtain a uniform adhesive agent layer composition. The solvent includes, for example, water, anhydrous ethanol, toluene, hexane, ethyl acetate, cyclohexane, heptane, butyl acetate, ethanol, methanol, xylene, isopropanol, and a mixture liquid of two or more of these. Subsequently, this adhesive agent layer composition is applied onto a surface of the backing layer (normally one of the surfaces), and thereafter, the solvent is dried and removed as necessary to form an adhesive agent layer. The product is further cut into a desired shape as necessary to obtain the patch of the present invention.

In addition, the method for producing the patch of the present invention may further include a step of applying the release liner on a surface of the adhesive agent layer opposite to the backing layer. The production method may be such that after the adhesive agent layer composition is first applied to one surface of the release liner to form the adhesive agent layer, the backing layer is applied onto the surface of the adhesive agent layer opposite to the release liner, and the product is cut into a desired shape as necessary to obtain the patch of the present invention. Moreover, the patch thus obtained may be sealed in the package container to obtain a packaged formulation as necessary.

The method for improving skin permeability of ropinirole and/or a pharmaceutically acceptable salt thereof of the present invention is a method in a ropinirole-containing patch comprising a backing layer and an adhesive agent layer, wherein
the adhesive agent layer containing:
at least one selected from the group consisting of ropinirole and a pharmaceutically acceptable salt thereof and
an adhesive agent,
the method comprising:
   a step of causing the adhesive agent layer to further contain a fatty acid metal salt having 7 or more carbon atoms.

The backing layer, the adhesive agent layer, the ropinirole and a pharmaceutically acceptable salt thereof, the adhesive agent, and the fatty acid metal salt having 7 or more carbon atoms are each as described above, including the preferable aspects.

The method for causing the adhesive agent layer to further contain the fatty acid metal salt according to the present invention includes, for example, a method including: mixing the ropinirole and/or a pharmaceutically acceptable salt thereof, the fatty acid metal salt, and the adhesive agent, as well as another drug, the absorption enhancer, the excipient, and the solvent, and the like as necessary; and molding the adhesive agent layer composition thus obtained to obtain the adhesive agent layer, as described in the above method for producing the patch of the present invention. In this way, it is possible to improve the skin permeability of ropinirole and/or a pharmaceutically acceptable salt thereof in the obtained ropinirole-containing patch.

### [Examples]

The present invention will be described more specifically below based on Examples, Comparative Examples, and Reference Examples; however, the present invention is not limited to Examples below. Note that the skin permeation test on patches obtained respectively in Examples, Comparative Examples, and Reference Examples were performed by a method described below.

### <Skin Permeation Test (In Vitro Hairless Mouse Skin Permeation Test)>

First, the skin of the trunk of a hairless mouse was peeled off and the adipose was removed, and the patch which had been cut into the size of 3.14 cm² and from which the release liner had been removed was applied to the epidermis side. This was set in a flow through-type Franz permeation test cell such that the dermis side came into contact with a receptor solution, and the cell was filled with the receptor solution (PBS). Subsequently, the receptor solution was sent at a flow rate of about 2.5 mL/hr while a warmed circulation water was caused to circulate on the outer periphery such that the receptor solution was kept at 32°C. Then, the receptor solution was sampled every 4 hours until 24 hours. The concentration of ropinirole (ropinirole hydrochloride) in the sampled receptor solution was measured by a high performance liquid chromatography, and the skin permeation amount of ropinirole per unit area of the adhesive agent layer was calculated in accordance with the following formula:
the skin permeation amount of ropinirole (µg/cm²) ={the concentration of ropinirole in the receptor solution (µg/mL) × the flow rate (mL) }/the patch area (cm²) to obtain the skin permeation amount per hour (skin permeation rate (µg/cm²/hr)). The maximum value of the skin permeation rate within 24 hours after the start of the application was obtained as a maximum skin permeation rate (Jmax, µg/cm²/hr). In addition, the cumulative skin permeation amount for 24 hours after the start of the application was obtained as a 24 hr cumulative skin permeation amount (µg/cm²). When the 24 hr cumulative skin permeation amount is 200 µg/cm² or more, the patch can be evaluated as being particularly excellent in skin permeability of ropinirole.

Moreover, the availability (%) of the drug (ropinirole) blended in each patch was obtained from the mass of ropinirole hydrochloride blended in each patch and the 24 hr cumulative skin permeation amount (µg/cm²) in accordance with the following formula:
the availability (%)={the 24 hr cumulative skin permeation amount (µg/cm²) × the patch area (cm²) × 100}/the mass of ropinirole hydrochloride blended in the patch. When the availability is 14% or more, the patch can be evaluated as being particularly excellent in skin permeability of ropinirole.

### (Example 1)

First, 15.0 parts by mass of ropinirole hydrochloride, 15.4 parts by mass of sodium oleate (corresponding to 1.0 mol relative to 1.0 mol of ropinirole hydrochloride in number of moles), 11.6 parts by mass of styrene-isoprene-styrene block copolymer (SIS), 7.7 parts by mass of polyisobutylene (PIB), 34.8 parts by mass of alicyclic saturated hydrocarbon resin, and 15.5 parts by mass of liquid paraffin were added to an appropriate amount of a solvent (water, methanol, and toluene), followed by mixing to obtain an adhesive agent layer composition. Subsequently, the adhesive agent layer composition thus obtained was applied onto a release liner (a film made of polyester and processed by release treatment) to have a thickness of 100 g/m², and the solvent was dried and removed to form an adhesive agent layer. A backing layer (a film made of polyester) was laminated on a surface of the adhesive agent layer thus obtained opposite to the release liner to obtain a patch in which the backing layer/the adhesive agent layer/the release liner were laminated in this order.

### (Examples 2 to 4, Comparative Examples 1 to 3)

Patches were obtained in the same manner as in Example 1 except that the composition of the adhesive agent layer composition became compositions shown in Tables 1 to 2 below.

The above-described skin permeation test was conducted on each of the patches obtained in Examples 1 to 4 and Comparative Examples 1 to 3. The results are shown in Tables 1 to 2 below together with the compositions (except for the solvent) of the respective adhesive agent layer compositions. Note that in Tables below, the numerical value in parentheses in the column of ropinirole hydrochloride indicates parts by mass in terms of free ropinirole.

**[Table 1]**

| | Example 1 | Example 2 | Example 3 | Example 4 |
|---|---|---|---|---|
| Adhesive agent layer composition [parts by mass] | | | | |
| Ropinirole hydrochloride (in terms of free form) | 15.0 (13.2) | 15.0 (13.2) | 15.0 (13.2) | 15.0 (13.2) |
| Sodium oleate (18 carbon atoms) | 15.4 | - | - | - |
| Sodium caprate (10 carbon atoms) | - | 9.8 | - | - |
| Sodium laurate (12 carbon atoms) | - | - | 11.2 | - |
| Potassium stearate (18 carbon atoms) | - | - | - | 16.3 |
| SIS | 11.6 | 12.5 | 12.3 | 11.4 |
| PIB | 7.7 | 8.4 | 8.2 | 7. 6 |
| Alicyclic saturated hydrocarbon resin | 34.8 | 37.6 | 36.9 | 34.4 |
| Liquid paraffin | 15.5 | 16.7 | 16.4 | 15.3 |
| Total | 100 | 100 | 100 | 100 |
| Maximum skin permeation rate [µg/cm²/hr] | 36.9 | 43.0 | 49.4 | 23.9 |
| 24 Hr cumulative skin permeation amount [µg/cm²] | 526.3 | 524.7 | 812.2 | 444.7 |
| Availability [%] | 35.1 | 35.0 | 54.1 | 29.6 |

**[Table 2]**

| | Comparative Example 1 | Comparative Example 2 | Comparative Example 3 |
|---|---|---|---|
| Adhesive agent layer composition [parts by mass] | | | |
| Ropinirole hydrochloride (in terms of free form) | 15.0 (13.2) | 15.0 (13.2) | 15.0 (13.2) |
| Sodium benzoate | 7.3 | - | - |
| Potassium sorbate (6 carbon atoms) | - | 7. 6 | - |
| Sodium lactate | - | - | 5.7 |
| SIS | 12.9 | 12.9 | 13.2 |
| PIB | 8. 6 | 8. 6 | 8.8 |
| Alicyclic saturated hydrocarbon resin | 38.9 | 38.7 | 39.7 |
| Liquid paraffin | 17.3 | 17.2 | 17.6 |
| Total | 100 | 100 | 100 |
| Maximum skin permeation rate [µg/cm²/hr] | 4.1 | 5.5 | 1.1 |
| 24 Hr cumulative skin permeation amount [µg/cm²] | 67.3 | 98.8 | 18.7 |
| Availability [%] | 4.5 | 6. 6 | 1.2 |

As is clear from the results shown in Table 1, it was confirmed that the patches of the present invention obtained using fatty acid metal salts having 7 or more carbon atoms (for example, Examples 1 to 4) were particularly excellent in the skin permeability of ropinirole. On the other hand, as is clear from the results shown in Table 2, it was confirmed that in the cases of using a metal salt of an aromatic acid (for example, Comparative Example 1), a metal salt of a fatty acid having less than 7 carbon atoms (for example, Comparative Example 2) , and a metal salt of another weak acid (for example, Comparative Example 3) in place of a fatty acid metal salt having 7 or more carbon atoms, the patches had low 24 hr cumulative skin permeation amounts and low availabilities, and were inferior to the patches of the present invention in the skin permeability of ropinirole.

### (Reference Examples 1 to 3)

In order to confirm that the excellent skin permeability in the patches obtained in Examples 1 to 4 was not attributable only to the generation of the free ropinirole and the fatty acid as a result of desalting of ropinirole hydrochloride and fatty acid metal salts, the effect of improving the skin permeability in the case where a fatty acid was combined with ropinirole hydrochloride and a desalting agent was studied. That is, patches were obtained in the same manner as in Example 1 except that the composition of the adhesive agent layer composition became compositions shown in Table 3 below.

The above-described skin permeation test was conducted on each of the patches obtained in Reference Examples 1 to 3. The results are shown in Table 3 below together with the compositions (except for the solvent) of the respective adhesive agent layer compositions.

**[Table 3]**

| | Reference Example 1 | Reference Example 2 | Reference Example 3 |
|---|---|---|---|
| Adhesive agent layer composition [parts by mass] | | | |
| Ropinirole hydrochloride (in terms of free form) | 15.0 (13.2) | 15.0 (13.2) | 15.0 (13.2) |
| Sodium hydroxide | 1. 62 | 1. 62 | 1. 62 |
| Oleic acid | - | 5 | - |
| Isostearic acid | - | - | 5 |
| SIS | 16.68 | 15.68 | 15.68 |
| PIB | 7.15 | 6.72 | 6.72 |
| Alicyclic saturated hydrocarbon resin | 40.5 | 38.07 | 38.07 |
| Liquid paraffin | 19.05 | 17.91 | 17.91 |
| Total | 100 | 100 | 100 |
| Maximum skin permeation rate [µg/cm²/hr] | 20.8 | 15.7 | 13.2 |
| 24 Hr cumulative skin permeation amount [µg/cm²] | 400.5 | 226.6 | 222.8 |
| Availability [%] | 26.7 | 15.1 | 14.9 |

As is clear from the results shown in Table 3, it was confirmed that although a skin permeability sufficient for a preparation was achieved also in the case of using sodium hydroxide, which has been conventionally used as a desalting agent for a salt of ropinirole (Reference Example 1), the skin permeability was not particularly improved but rather tended to decrease when a fatty acid having 7 or more carbon atoms was added to this (Reference Examples 2 to 3). Hence, it was confirmed that the excellent skin permeability in the patch of the present invention was not attributable only to the generation of the free ropinirole and the fatty acid by the desalting reaction of the ropinirole hydrochloride and the fatty acid metal salt having 7 or more carbon atoms.

### (Examples 5 to 7)

Patches were obtained in the same manner as in Example 1 except that the composition of the adhesive agent layer composition became compositions shown in Table 4 below. In Table 4, adhesive agents were as follows:
MAS-811B: an acrylic-based adhesive agent having substantially no functional group (manufactured by CosMED Pharmaceutical Co.Ltd.),
Duro-Tak-87-4287: a OH group-containing acrylic-based adhesive agent having substantially no carboxy group (manufactured by Henkel AG & Co. KGaA), and
Bio-PSA-7-4202: Bio-PSA-7-4202, silicone-based adhesive agent (manufactured by Dow Corning Corp.) (hereinafter, the same applies to Table 6.).

The above-described skin permeation test was conducted on each of the patches obtained in Examples 5 to 7. The results are shown in Table 4 below together with the compositions (except for the solvent) of the respective adhesive agent layer compositions.

**[Table 4]**

| | Example 5 | Example 6 | Example 7 |
|---|---|---|---|
| Adhesive agent layer composition [parts by mass] | | | |
| Ropinirole hydrochloride (in terms of free form) | 15.0 (13.2) | 15.0 (13.2) | 15.0 (13.2) |
| Potassium stearate (18 carbon atoms) | 16.3 | 16.3 | 16.3 |
| MAS-811B | 68.7 | - | - |
| Duro-Tak-87-4287 | - | 68.7 | - |
| BiO-PSA-7-4202 | - | - | 68.7 |
| Total | 100 | 100 | 100 |
| Maximum skin permeation rate [µg/cm²/hr] | 24.1 | 19.4 | 10.4 |
| 24 Hr cumulative skin permeation amount [µg/cm²] | 458.9 | 370.9 | 211.1 |
| Availability [%] | 30.6 | 24.7 | 14.1 |

As is clear from the results shown in Table 4, it was confirmed that in the case where an acrylic-based adhesive agent or a silicone-based adhesive agent were used as the adhesive agent in place of a rubber-based adhesive agent (for example, SIS and PIB) as well, the patches of the present invention (for example, Examples 5 to 7) obtained using a fatty acid metal salt having 7 or more carbon atoms were excellent in the skin permeability of ropinirole as in the case of using a rubber-based adhesive agent (for example, Example 4).

### (Example 8, Comparative Examples 4 to 5)

Patches were obtained in the same manner as in Example 1 except that the composition of the adhesive agent layer composition became compositions shown in Table 5 below.

The above-described skin permeation test was conducted on each of the patches obtained in Example 8 and Comparative Examples 4 to 5. The results are shown in Table 5 below together with the compositions (except for the solvent) of the respective adhesive agent layer compositions.

**[Table 5]**

| | Example 8 | Comparative Example 4 | Comparative Example 5 |
|---|---|---|---|
| Adhesive agent layer composition [parts by mass] | | | |
| Ropinirole hydrochloride (in terms of free form) | 15.0 (13.2) | 15.0 (13.2) | 15.0 (13.2) |
| Sodium caprylate (8 carbon atoms) | 8.4 | - | - |
| Sodium adipate (6 carbon atoms) | - | 9. 6 | - |
| Sodium gluconate (6 carbon atoms) | - | - | 11 |
| SIS | 12.77 | 12.57 | 12.33 |
| PIB | 8.51 | 8.38 | 8.22 |
| Alicyclic saturated hydrocarbon resin | 38.3 | 37.7 | 37 |
| Liquid paraffin | 17.02 | 16.75 | 16.45 |
| Total | 100 | 100 | 100 |
| Maximum skin permeation rate [µg/cm²/hr] | 26.2 | 3. 6 | 0.8 |
| 24 Hr cumulative skin permeation amount [µg/cm²] | 438 | 53 | 12 |
| Availability [%] | 29.2 | 3.5 | 0.8 |

As is clear from the results shown in Table 5, it was confirmed that the patch of the present invention (for example, Example 8) obtained using a fatty acid metal salt having 7 or more carbon atoms was particularly excellent in the skin permeability of ropinirole. On the other hand, it was confirmed that in the case of using a metal salt of a fatty acid having less than 7 carbon atoms (for example, Comparative Examples 4 to 5) in place of a fatty acid metal salt having 7 or more carbon atoms, the patches had low 24 hr cumulative skin permeation amounts and low availabilities, and were inferior to the patch of the present invention in the skin permeability of ropinirole.

### (Examples 9 to 14)

Patches were obtained in the same manner as in Example 1 except that the composition of the adhesive agent layer composition became compositions shown in Table 6 below.

The above-described skin permeation test was conducted on each of the patches obtained in Examples 9 to 14. The results are shown in Table 6 below together with the compositions (except for the solvent) of the respective adhesive agent layer compositions.

**[Table 6]**

| | Example 9 | Example 10 | Example 11 | Example 12 | Example 13 | Example 14 |
|---|---|---|---|---|---|---|
| Adhesive agent layer composition [parts by mass] | | | | | | |
| Ropinirole hydrochloride (in terms of free form) | 15.0 (13.2) | 15.0 (13.2) | 15.0 (13.2) | 15.0 (13.2) | 15.0 (13.2) | 15.0 (13.2) |
| Sodium caprylate (8 carbon atoms) | 8.4 | 8.4 | 8.4 | 8.4 | 8.4 | 42 |
| SIS | 21.28 | - | - | - | - | 7.17 |
| PIB | - | 76.6 | - | - | - | 4.78 |
| Alicyclic saturated hydrocarbon resin | 38.3 | - | - | - | - | 21.5 |
| Liquid paraffin | 17.02 | - | - | - | - | 9.55 |
| MAS-811B | - | - | 76.6 | - | - | - |
| Duro-Tak-87-4287 | - | - | - | 76.6 | - | - |
| BiO-PSA-7-4202 | - | - | - | - | 76.6 | - |
| Total | 100 | 100 | 100 | 100 | 100 | 100 |
| Maximum skin permeation rate [µg/cm²/hr] | 24.8 | 46.9 | 52.8 | 19.7 | 52.3 | 58.4 |
| 24 Hr cumulative skin permeation amount [µg/cm²] | 314 | 452 | 586 | 355 | 830 | 820 |
| Availability [%] | 20.9 | 30.1 | 39.1 | 23.7 | 55.3 | 54.7 |

As is clear from the results shown in Table 6, it was confirmed that in the case of using only SIS or PIB as a rubber-based adhesive agent (for example, Examples 9 to 10) and the case of using an acrylic-based adhesive agent or a silicone-based adhesive agent in place of a rubber-based adhesive agent (for example, Examples 11 to 13) as well, the patches of the present invention obtained using a fatty acid metal salt having 7 or more carbon atoms were excellent in the skin permeability of ropinirole as in the case of using a rubber-based adhesive agent (for example, Example 8). In addition, it was confirmed that even when the content of the fatty acid metal salt having 7 or more carbon atoms was 5.0 mol relative to 1.0 mol in content in terms of free ropinirole, the patch of the present invention (for example, Example 14) was excellent in the skin permeability of ropinirole.

### [Industrial Applicability]

As described above, the present invention makes it possible to provide a ropinirole-containing patch that is excellent in skin permeability of ropinirole and/or a pharmaceutically acceptable salt thereof and a method for improving skin permeability of ropinirole and/or a pharmaceutically acceptable salt thereof.

## Claims

1. A ropinirole-containing patch comprising a backing layer and an adhesive agent layer, wherein
the adhesive agent layer containing:
at least one selected from the group consisting of ropinirole and a pharmaceutically acceptable salt thereof;
a fatty acid metal salt having 7 or more carbon atoms; and
an adhesive agent.

2. The ropinirole-containing patch according to claim 1, wherein
the fatty acid metal salt is a fatty acid alkali metal salt.

3. The ropinirole-containing patch according to claim 1 or 2, wherein
the fatty acid metal salt is a metal salt of at least one fatty acid selected from the group consisting of caprylic acid, oleic acid, lauric acid, stearic acid, capric acid, myristic acid, and palmitic acid.

4. The ropinirole-containing patch according to any one of claims 1 to 3, wherein
the adhesive agent is at least one selected from the group consisting of a rubber-based adhesive agent, an acrylic-based adhesive agent having no carboxy group, and a silicone-based adhesive agent.

5. The ropinirole-containing patch according to any one of claims 1 to 4, wherein
a content of the fatty acid metal salt is 0.1 to 45% by mass relative to a total mass of the adhesive agent layer.

6. The ropinirole-containing patch according to any one of claims 1 to 5, wherein
in the adhesive agent layer, a content of the fatty acid metal salt is 0.1 to 5.0 mol relative to 1.0 mol of the at least one selected from the group consisting of ropinirole and a pharmaceutically acceptable salt thereof in content in terms of free ropinirole.

7. The ropinirole-containing patch according to any one of claims 1 to 6, wherein
a content of the at least one selected from the group consisting of ropinirole and a pharmaceutically acceptable salt thereof in terms of free ropinirole is 5 to 30% by mass relative to a total mass of the adhesive agent layer.

8. A method for improving skin permeability of at least one selected from the group consisting of ropinirole and a pharmaceutically acceptable salt thereof in a ropinirole-containing patch comprising a backing layer and an adhesive agent layer, wherein
the adhesive agent layer containing:
the at least one selected from the group consisting of ropinirole and a pharmaceutically acceptable salt thereof and
an adhesive agent,
the method comprising:
a step of causing the adhesive agent layer to further contain a fatty acid metal salt having 7 or more carbon atoms .
